# EUROPEAN PATENT SPECIFICATION

(11) **EP 0 605 371 B1**
(45) Date of publication and mention of the grant of the patent: **04.06.1997**
(21) Application number: 93830535.6
(22) Date of filing: 30.12.1993
(51) Int. Cl.: A61K 31/40

(54) **Use of melatonin for the manufacture of a medicament for the treatment of scleroderma and sarcoidosis**
Verwendung von Melatonin zur Herstellung eines Arzneimittels zur Behandlung von Scleroderma und der Sarcoidosis
Utilisation de la mélatonine pour la fabrication d'un médicament pour le traitement de la sclérose systémique et de la sarcoidose

(30) Priority: 30.12.1992 IT FI920241
(43) Date of publication of application: 06.07.1994
(73) Proprietor: Cagnoni, Mario, I-50139 Firenze (IT)
(72) Inventor: Cagnoni, Mario, I-50139 Firenze (IT)
(74) Representative: Mannucci, Gianfranco, Dott.-Ing.

(56) References cited:
- EP-A- 0 513 702
- MEDICAL HYPOTHESES, vol.5, no.4, 1979 pages 403 - 414 S.C.CUNNANE ET AL. 'The pineal and regulation of fibrosis: Pinealectomy as a model of primary biliary cirrhosis: Role of melatonin and prostaglandins in fibrosis and regulation of T lymphocytes'
- ANNALES OF THE RHEUMATIC DISEASES, vol.51, no.2, 1992 pages 286 - 288 E.CARWILE LEROY 'A brief overview of the pathogenesis of scleroderma (systemic sclerosis)'
- ROBERT BERKOW ET AL. 'The merck manual' , MERCK RESEARCH LABORATORIES , RAHWAY N.J. USA * page 272 - page 275 *
- MEDICAL HYPOTHESES, vol.7, no.9, 1981 pages 1211 - 1220 DAVID F. HORROBIN 'Loss of delta-6-desaturase activity as a key factor in aging'
- ROBERT BERKOW ET AL.: 'The merck manual', MERCK RESEARCH LABORATORIES, RAHWAY, N.J., US

## Description

Melatonin (5-methoxy-N-acetyltryptamine) is synthesized by the pineal body. It results from the hydroxylation and decarboxylation of tryptophan to 5-hydroxytryptamine (serotonin) and the subsequent N-acetylation of serotonin by the enzyme N-acetyltransferase.

Melatonin is secreted rhythmically into the circulation and distributed to the target cells, to which it binds at specific sites. The circadian rhythm of melatonin levels is synchronized with the alternation of lightness and darkness.

The activity of the enzyme N-acetyltransferase increases 30 to 70 times during the nocturnal hours and consequently the maximum levels of melatonin are found between 24.00 and 4.00 hours.

The synthesis of melatonin is promoted by noradrenergic stimulation by sympathetic postganglionic nerve fibers which, in rodents at least, terminate at the beta₁ receptors (cf.: Klein, D.C. "Melatonin and the pineal". 1985. Eds. D. Evered and S. Clark (Pitman, London) 38-56; and Cowen, P.J., Fraser, S. et al. 1983. Brit. J. Clin. Pharmacol. 19, 579-581).

Melatonin is metabolized in the brain and the liver and excreted in the urine; it acts as a neurotransmitter and neuromodulator; it also plays a major role in interactions between the neuroendocrine and immune systems.

No signs of toxicity have ever been observed after the administration of melatonin (cf.: Norlund, J., Lerner, A.B. 1977. J. Clin. Endocrinol. Metab. 45, 768-774) and there are therefore no contraindications to its use in the treatment of man.

Numerous studies have been conducted, some of them in healthy volunteers, to evaluate the effect of melatonin in synchronizing the sleep-wake pattern (cf. Anton-Tay, F., Diaz, J.L., et al. 1971. Life Science 10, 841-850; and Cramer, H., Rudolph, J., et al. 1974. Ad. Biochem. Psycho-pharmacol. 11, 187-191).

Treatment with melatonin has been tested in a large number of different pathological conditions. Very high doses have been used in some cases: for example, daily doses of up to 6 g administered to patients with Parkinson's disease had no adverse effects, but also no demonstrable advantages.

Many clinical and experimental reports (cf.: Bartsch, C., Bartsch, H., et al. 1989. Cancer 64, 426-433; Raikhlin, N.T., Kventoy, I.M., et al. 1980. Klin. Med. (Mosk) 58-77-79; Batsch, C., Bartsch, H., et al. 1981. J. Neural. Transm. 52, 281-294; Tamarkin, L., Danfort, D., et al. 1982. Science 216, 1003-1005; Bartsch, C., Bartsch, H., et al. 1991. Cancer 67, 1681-1684) have suggested that there is a link between the pineal body and neoplastic disease and, more particularly, have demonstrated that melatonin has antiproliferative activity (cf.: Marabini, S., Carossino, A.M., Cagnoni, M., 1992, Med. Sci. Res. 20, 551-553; Blask, D.E., 1984. in: Reiter, "The pineal gland". pp.253-284. Raven Press, NY; Smythe, S.A., Stuart, M.C., et al. 1974. Experientia 30, 1356-1357).

The therapeutic effect of melatonin has also been evaluated in cancer patients who were no longer responsive to conventional therapies. The results were encouraging insofar as the patients' general condition showed improvement and the disease was temporarily stabilized (cf.: Lissoni, P., Barni, S., et al. 1987. Tumori 73, 475-480; Lissoni, P., Viviani, S., et al. 1986. Cancer 57, 837-842; Lissoni, P., Barni, S., et al. 1989 Eur. J. Cancer Clin. Oncol. 23 (5), 789-795).

The invention is based on the use of melatonin (5-methoxy-N-acetyltryptamine) to treat systemic scleroaerma and sarcoidosis and also other diseases of a fibroplastic nature involving fibrotic manifestations, such as chronic cirrhogenous liver disease, at a stage preceding fibrosis

In S.C. Cunnane et al "The pineal and regulation of fibrosis: pinealectomy as a model of primary biliary cirrhosis: role of melatonin and prostaglandinis in fibrosis and regulation of T-Lymphocytes" (published in "Medical Hypotheses, p. 403-414, 1979) it is suggested that melatonin, PGE1 and TXA2 are important in the regulation of fibrosis in situation such as "collagen" diseases. S.C. Cunnane et al suggest to raise PGE1 and TXA2 levels by administering different compounds, in order to treat a large variety of diseases.

The subject of the invention is the use of melatonin for the manufacture of a medicament for the treatment of fibrosant diseases at a stage preceding fibrosclerosis. Particularly, according to the invention melatonin may be used for the production of medicaments for the treatment of systemic sleroderma and sarcoidosis. For the treatment of these diseases, single daily doses of 15-25 mg, and preferably 20 mg are manufactured.

Treatment with nelatonin was administered to ambulant patients with various forms of systemic scleroderma, in various stages of the disease, and to patients with sarcoidosis.

All the patients were selected because they exhibited a demonstrable reduction in pulmorary function, on the basis that in both diseases this reduction evolves into the irreversible condition of pulmonary fibrosis after passing through stages dominated by a variety of inflammatory manifestations.

The use of melatonin for its modulating effect on the neuro-immuno-endocrine system seems to be particularly justified in the stages characterized by intense inflammatory activity which precede the appearance and evolution of the fibrosclerotic process.

Fibrosis, according to the histopathological definition, always constitutes a terminal stage and is therefore not amenable to treatment. By contrast, the processes that precede it, which can be designated by a term that stresses their dynamic aspects, such as "sclerosing", can probably be treated to good effect.

The effect of melatonin can be demonstrated with the aid of lung function tests, determination of the blood gases and radiological investigation.

The patients and methods were as follows:

Ten patients with systemic scleroderma in an active phase and four patients with pulmonary sarcoidosis were selected.

The clinical characteristics of the patients with systemic scleroderma and the level of activity and severity of the disease were assessed with the aid of a skin score (cf. annexe) and information obtained from lung function tests, a chest X-ray, pulmonary CAT and numerous laboratory tests (antinuclear antibodies (A), A to the extractable nuclear antigen Scl-70, antinucleolar A, anti-DNA A, antimicrosome A, anticentromeric A, ESR, ACE, CRP, gamma globulins).

To assess the effects of the treatment, the skin-score, ESR, ACE, CRP were determined monthly, the lung function tests were performed every 2 months, and chest X-ray and/or thoracic CAT carried out at the end of a 4-month course of treatment.

All the patients with sarcoidosis exhibited a definitive impairment of pulmonary function and were between stages II and III.

The diagnosis was made on the basis of histological tests performed on biopsy samples, the BAL for the T-lymphocyte count and scintigraphy with gallium-67. Overall lung function tests, a chest X-ray and numerous laboratory tests (ACE, blood calcium, urinary calcium, ESR, etc.) were also performed.

The effects of the treatment were evaluated monthly on the basis of any changes in lung function and ACE levels and at the end of the four-month period on the basis of any changes in the radiological findings.

When the clinical evaluation had been completed and after all the laboratory tests had been performed, the patients with systemic scleroderma and sarcoidosis received in a daily dose of 20 mg taken by mouth as a single dose between 18.00 and 20.00 hours. This timing was chosen with the aim of interfering as little as possible with the circadian rhythm of endogenous melatonin secretion. In fact, evening administration produces an increase in blood melatonin levels at a time when the secretory activity of the cells producing the hormone has already commenced.

Two patients with systemic scleroderma were already being treated with hydroxychloroquine in a daily dose of 5 mg per kg; this treatment was continued in association with melatonin.

The treatment with hydroxychloroquine had been started at least 4 months before that with melatonin. The clinical investigations and laboratory tests performed at the start of the combined treatment therefore served both as a control on the treatment with hydroxychloroquine and to provide the base values for the tests performed during the treatment with melatonin.

The other patients with systemic scleroderma and those with sarcoidosis received only melatonin.

None of the patients exhibited any adverse effects necessitating suspension of the treatment.

The results obtained are reported below.

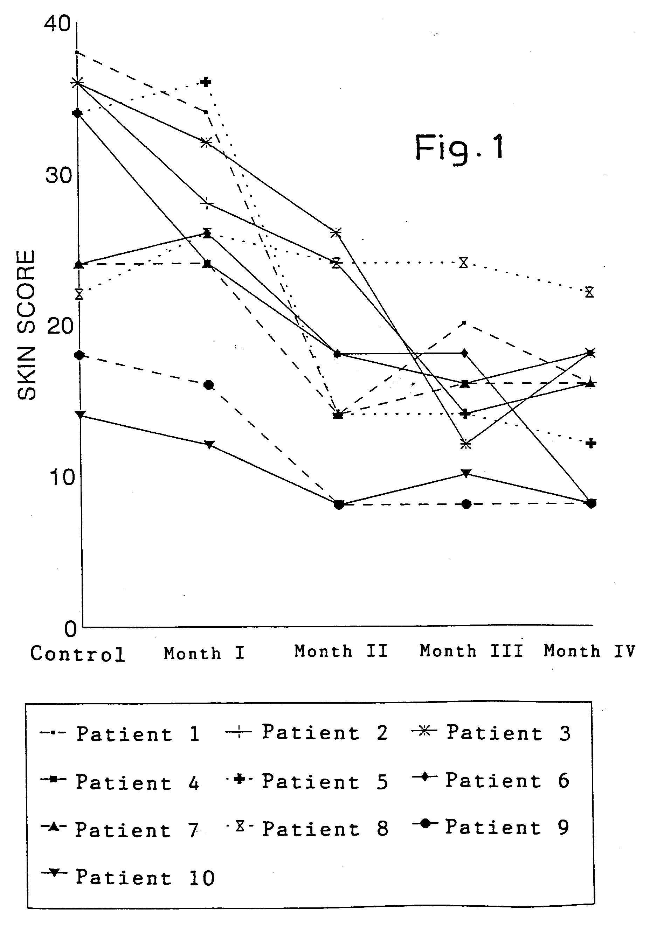

In the group of ten patients with systemic sarcoidosis, the treatment with 20 mg melatonin per day for four months produced highly positive results, particularly in terms of the skin score (FIG.1) and the lung function tests.

The latter showed a significant improvement in nine of the ten patients. This was accompanied by a marked subjective reduction in the respiratory disturbances previously reported.

The ACE values at the start of treatment were close to the lower limit of normal in seven patients and just above the maximum normal values in the other three. The normal range for fluorimetric determination performed in the laboratory is 8 ± 2 U/l.

No significant changes were found in the CRP or gamma globulin values, which were, in fact, higher than normal in only two cases (Nos 7 and 8).

The ESR was initially higher than normal in all cases and throughout the course of treatment with melatonin it showed variations compatible with the characteristic episodic course of the sclerodermatous disease. It was not possible to demonstrate that the hormone treatment had an effect on this parameter. Studies involving a larger number of patients and/or a longer period of treatment would probably yield information of value in this respect.

After 4 months of treatment, a chest X-ray was performed in all the patients. No significant changes were found in any of the patients. However, it should be mentioned that in eight of the ten patients the radiological changes were extremely modest, even where there was severe impairment of the respiratory parameters. It should also be stressed that in this type of disease while radiological investigation can confirm suspected pulmonary fibrosis there is no precise correlation with the extent and severity of the lesions.

In the two cases in which the changes were more marked (Nos 8 and 10, both treated simultaneously with hydroxychloroquine and melatonin), the follow-up X-ray and CAT performed at the end of the 4-month course of treatment failed to reveal any significant changes. In one of these two cases (No.8), however, the lung function tests had shown a slight improvement accompanied by a reduction in the subjective symptoms.

The results obtained with melatonin in the patients with pulmonary sarcoidosis, all between stages II and III, were particularly satisfactory.

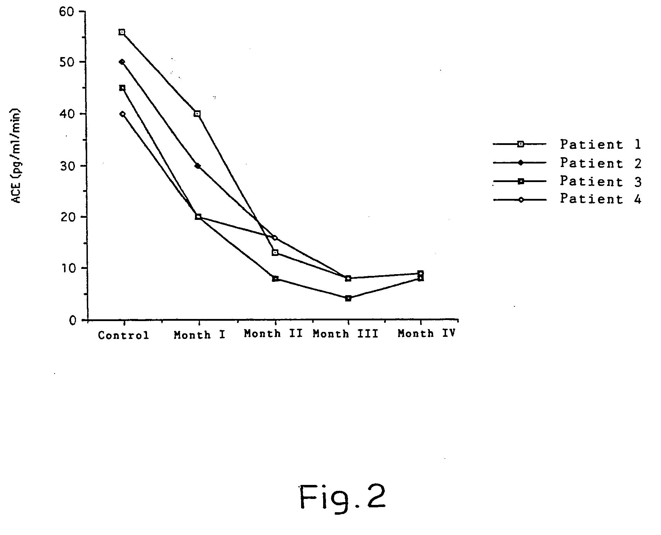

The monthly determination of ACE showed in all cases that the values gradually returned to normal (FIG.2).

The results of the lung function tests were also highly significant. Initially, all the patients exhibited restrictive signs with severe alteration of the diffusion of gases and resting hypoxemia. At the end of the four-month course of treatment, the findings in three patients had returned to normal and the fourth patient showed marked improvement.

The radiological investigation showed a definitive reduction in the changes affecting the hilum and the pulmonary parenchyma. Thoracic CAT was performed in one case at both the beginning and the end of the treatment. This confirmed the improvement seen on the X-ray.

Before the treatment, the BAL test showed lymphocyte percentages in excess of 30% in all the patients. At the end of the four-month course of treatment, the lymphocyte count had fallen by 70% on average. However, the CD4/CD8 ratio had returned to normal after only two months' treatment.

In conclusion, the results obtained with treatment based on melatonin in patients with fibroplastic diseases such as, and in particular, systemic scleroderma and pulmonary sarcoidosis, showed a significant improvement in the disease parameters investigated.

## Claims

1. The use of melatonin (5-methoxy-N-acetyltryptamine) for the manufacture of a medicament for the treatment of fibrosant diseases at a stage preceding fibrosclerosis.

2. The use of melatonin (5-methoxy-N-acetyltryptamine) according to claim 1, for the manufacture of a medicament for the treatment of systemic scleroderma.

3. The use of melatonin (5-methoxy-N-acetyltryptamine) according to claim 1, for the manufacture of a medicament for the treatment of sarcoidosis.

4. The use of melatonin as claimed in one of claims 1, 2 or 3, wherein single daily doses of the order of 15 to 25 mg are manufactured.

5. The use of melatonin as claimed in one of claims 1, 2 or 3, wherein single daily doses of the order of approximately 20 mg are manufactured.

## Patentansprüche

1. Verwendung von Melatonin (5-Methoxy-N-Acetyltryptamin) für die Herstellung eines Medikaments für die Behandlung von fibrosierenden Störungen in einem Vorstadium von Fibrosklerose.

2. Verwendung von Melatonin (5-Methoxy-N-Acetyltryptamin) gemäß Anspruch 1 für die Herstellung eines Medikaments für die Behandlung von systemischer Sklerodermie.

3. Verwendung von Melatonin (5-Methoxy-N-Acetyltryptamin) gemäß Anspruch 1 für die Herstellung eines Medikaments für die Behandlung von Sarkoidose.

4. Verwendung von Melatonin nach einem der Ansprüche 1, 2 oder 3, bei der einzelne Tagesdosen in der Größenordnung von 15 bis 25 mg hergestellt werden.

5. Verwendung von Melatonin nach einem der Ansprüche 1, 2 oder 3, bei der einzelne Tagesdosen in der Größenordnung von ca. 20 mg hergestellt werden.

## Revendications

1. Utilisation de la mélatonine (5-méthoxy-N-acétyltryptamine) pour la fabrication d'un médicament destiné au traitement d'affections fibrosantes à un stade précédant la fibrosclérose.

2. Utilisation de la mélatonine (5-méthoxy-N-acétyltryptamine) selon la revendication 1, pour la fabrication d'un médicament destiné au traitement de la sclérodermie systémique.

3. Utilisation de la mélatonine (5-méthoxy-N-acétyltryptamine) selon la revendication 1, pour la fabrication d'un médicament destiné au traitement de la sarcoïdose.

4. Utilisation de la mélatonine selon l'une quelconque des revendications 1, 2 ou 3, dans laquelle on fabrique des doses unitaires journalières de l'ordre de 15 à 25 mg.

5. Utilisation de la mélatonine selon l'une quelconque des revendications 1, 2 ou 3, dans laquelle on fabrique des doses journalières unitaires de l'ordre d'environ 20 mg.
